# EUROPÄISCHE PATENTSCHRIFT

(11) **EP 1 476 527 B1**
(45) Veröffentlichungstag und Bekanntmachung des Hinweises auf die Patenterteilung: **11.04.2007**
(21) Anmeldenummer: 03706463.1
(22) Anmeldetag: 10.02.2003
(51) Int. Cl.: C11B 9/00, C07C 47/21, A61Q 13/00

(54) **VERWENDUNG VON HEXENAL-DERIVATEN ALS RIECHSTOFFE**
USE OF HEXENAL DERIVATIVES AS PERFUMES
UTILISATION DE DERIVES D'HEXENAL COMME PARFUMS

(30) Priorität: 19.02.2002 DE 10206771
(43) Veröffentlichungstag der Anmeldung: 17.11.2004
(73) Patentinhaber: KAO CORPORATION, Tokyo 103-8210 (JP)
(72) Erfinder: MARKERT, Thomas, 40789 Monheim (DE); SPEITKAMP, Marc, 40476 Düsseldorf (DE); RITTLER, Frank, 40479 Düsseldorf (DE)
(74) Vertreter: HOFFMANN EITLE
(86) Internationale Anmeldenummer: PCT/EP2003/001273
(87) Internationale Veröffentlichungsnummer: WO 2003/070864

(56) Entgegenhaltungen:
- FR-A- 1 409 326
- FR-A- 2 430 402
- US-A- 3 704 714
- US-A- 4 010 207
- F. ANSELL ET AL: "The synthesis and reactions of branched-chain hydrocarbons. Part X . The rearrangement of alpha-ethynyl alcohols to unsaturated carbonyl compounds." JOURNAL OF THE CHEMICAL SOCIETY, 1956, Seite 911-917 XP002242815 CHEMICAL SOCIETY. LETCHWORTH.; GB in der Anmeldung erwähnt

## Beschreibung

### Gebiet der Erfindung

Die vorliegende Erfindung betrifft die Verwendung von Hexenal-Derivaten spezieller Struktur als Riechstoffe.

### Stand der Technik

Viele natürliche Riechstoffe stehen, gemessen am Bedarf, in völlig unzureichender Menge zur Verfügung. Beispielsweise sind zur Gewinnung von 1 kg Rosenöl 5000 kg Rosenblüten notwendig. Die Folgen sind eine sehr stark limitierte Weltjahresproduktion sowie ein hoher Preis. Es ist daher klar, dass die Riechstoffindustrie einen ständigen Bedarf an neuen Riechstoffen mit interessanten Duftnoten hat. Einerseits kann dadurch die Palette der natürlich verfügbaren Riechstoffe ergänzt werden, andererseits ist es dadurch möglich, die notwendigen Anpassungen an wechselnde modische Geschmacksrichtungen vornehmen zu können. Darüber hinaus wird es auf diese Weise möglich, den ständig steigenden Bedarf an Geruchsverbesserern für Produkte des täglichen Bedarfs wie Kosmetika und Reinigungsmittel decken zu können.

Im Übrigen besteht generell ein ständiger Bedarf an synthetischen Riechstoffen, die sich günstig und mit gleichbleibend hoher Qualität herstellen lassen und die originelle olfaktorische Eigenschaften haben. Insbesondere sollen sie angenehme, möglichst naturnahe und qualitativ neuartige Geruchsprofile von ausreichender Intensität besitzen und in der Lage sein, den Duft von kosmetischen und Verbrauchsgütern vorteilhaft zu beeinflussen. Mit anderen Worten: es besteht ein ständiger Bedarf an Verbindungen, die charakteristische neue Geruchsprofile bei gleichzeitig hoher Haftfestigkeit, Geruchsintensität und Strahlkraft aufweisen.

M.F.Ansell, J.W. Hancock and W.J. Hickinbottom, J.Chem.Soc. 1956, Seite 911 berichten, daß sie 5-Methyl-3-isobutyl-2(3)-hexenal neben den isomeren Ketonen bei der Rupe-Umlagerung von 3-Isobutyl-5-methyl-hex-1-in-3-ol in geringen Mengen erhalten hatten. Über geruchliche Eigenschaften oder die Eignung der Verbindungen als Riechstoffe ist nichts offenbart.

### Beschreibung der Erfindung

Es wurde gefunden, dass die Verbindungen der allgemeinen Formel (I) die oben genannten Forderungen in jeder Hinsicht ausgezeichnet erfüllen und in vorteilhafter Weise als Riechstoffe mit unterschiedlichen nuancierten Geruchsnoten mit guter Haftfestigkeit eingesetzt werden können.

Gegenstand der vorliegenden Erfindung ist zunächst die Verwendung von Hexenal-Derivaten der allgemeinen Struktur (I) 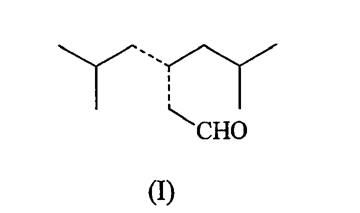 worin eine der strichlierten Bindungen eine C-C-Einfachbindung und die andere eine C=C-Doppelbindung bedeutet, mit der Maßgabe, daß die C=C-Doppelbindung Z- oder E-konfiguriert sein kann, sofern sie sich in Position C3/4 befindet, als Riechstoffe.

Insgesamt beinhaltet Formel (I) drei chemische Individuen, nämlich
- 5-Methyl-3-isobutyl-2-hexenal (I-a)
- 5-Methyl-3-isobutyl-3-hexenal mit E-konfigurieter C=C-Doppelbindung (I-b)
- 5-Methyl-3-isobutyl-3-hexenal mit Z-konfigurieter C=C-Doppelbindung (I-c)

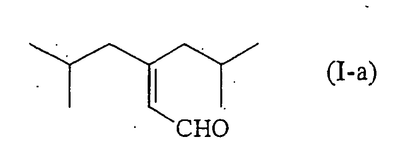

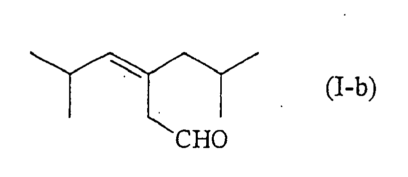

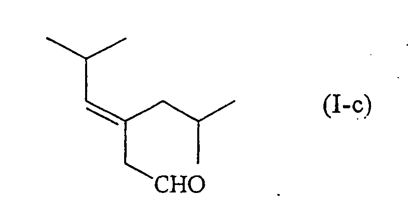

Bei der erfindungsgemäßen Verwendung können die Verbindungen (I-a), (I-b) und (I-c) einzeln oder im Gemisch miteinander eingesetzt werden. Besonders bevorzugt ist die Verwendung von Gemischen der Verbindungen (I-a), (I-b) und (I-c).

In einer weiteren Ausführungsform betrifft die Erfindung die Verwendung von einer oder mehreren Verbindungen der oben näher bezeichneten allgemeinen Struktur (I) als Riechstoff-Konzentrate.

Die erfindungsgemäßen Verbindungen (I), zeichnen sich durch eine Geruchscharakteristik aus, in der Citrus-Noten dominieren. Sie weisen eine ausgezeichnete Stabilität in Rezepturen der Kosmetik und Gebrauchsparfümerie auf.

Die Herstellung der Verbindungen (I) kann nach bekannten Syntheseverfahren der organischen Chemie erfolgen. Eine präparativ attraktive Möglichkeit für die Herstellung eines Gemisches der Verbindungen (I-a), (I-b) und (I-c) kann dem Beispielteil entnommen werden.

In Parfüm-Kompositionen verstärken die Verbindungen (I) die Harmonie und die Ausstrahlung sowie die Natürlichkeit und auch die Haftung, wobei die Dosierung unter Berücksichtigung der übrigen Bestandteile der Komposition auf die jeweils angestrebte Duftnote abgestimmt wird.

Dass die Verbindungen (I) die oben genannten Duftnoten aufweisen, war nicht vorhersehbar und ist damit eine weitere Bestätigung für die allgemeine Erfahrung, dass die olfaktorischen Eigenschaften bekannter Riechstoffe keine zwingenden Rückschlüsse auf die Eigenschaften strukturverwandter Verbindungen zulassen, weil weder der Mechanismus der Duftwahrnehmung noch der Einfluss der chemischen Struktur auf die Duftwahrnehmung hinreichend erforscht sind und somit also normalerweise nicht vorhergesehen werden kann, ob ein geänderter Aufbau bekannter Riechstoffe überhaupt zur Änderung der olfaktorischen Eigenschaften führt und ob diese Änderungen vom Fachmann positiv oder negativ beurteilt werden.

Die Verbindungen der Formel (I) eignen sich aufgrund ihres Geruchsprofils insbesondere auch zur Modifizierung und Verstärkung bekannter Kompositionen. Hervorgehoben werden soll insbesondere ihre ausserordentliche Geruchsstärke, die ganz allgemein zur Veredelung der Komposition beiträgt.

Die Verbindungen der Formel (I) lassen sich mit zahlreichen bekannten Riechstoffingredienzien, beispielsweise anderen Riechstoffen natürlichen, synthetischen oder partial-synthetischen Ursprungs, ätherischen Ölen und Pflanzenextrakten kombinieren. Die Palette der natürlichen Riechstoffe kann dabei sowohl leicht-, als auch mittel- und schwerflüchtige Komponenten umfassen. Die Palette der synthetischen Riechstoffe kann Vertreter aus praktisch allen Stoffklassen umfassen.

Beispiele für geeignete Substanzen, mit denen die Verbindungen (I) kombiniert werden können sind insbesondere:
(a) Naturprodukte wie Baummoos-Absolue, Basilikumöl, Agrumenöle wie Bergamotteöl, Mandarinenöl, usw., Mastix-Absolue, Myrtenöl, Palmarosaöl, Patchouliöl, Petitgrainöl, Wermutöl, Myrrheöl, Olibanumöl, Cedernholzöl, Sandelholzöl, ostindisch, Guajakholzöl, Cabreuva,
(b) Alkohole wie Farnesol, Geraniol, Citronellol, Linalool, Nerol, Phenylethylalkohol, Rhodinol, Zimtalkohol, Sandalore [3-Methyl-5-(2.2.3-trimethylcyclopent-3-en-1-yl)pentan-2-ol], Sandela [3-Isocamphyl-(5)-cyclohexanol], Mugetanol,
(c) Aldehyde wie Citral, Helional^{R}, alpha-Hexylzimtaldehyd, Hydroxycitronellal, Lilial^{R} [p-tert.-Butyl-α-methyldihydrozimtaldehyd], Methylnonylacetaldehyd,
(d) Ketone wie Allylionon, α-Ionon, β-Ionon, Isoraldein, Methylionon, Nootkaton, Calone, α-, β- und γ-Irone, Damascone,
(e) Ester wie Allylphenoxyacetat, Benzylsalicylat, Cinnamylpropionat, Citronellylacetat, Decylacetat, Dimethylbenzylcarbinylacetat, Ethylacetoacetat, Hexenylisobutyrat, Linalylacetat, Methyldihydrojasmonat, Vetiverylacetat, Cyclohexylsalicylat, Isobomylisobutyrat, Evernyl,
(f) Lactone wie gamma-Undecalacton, 1-Oxaspiro[4.4]nonan-2-on, Cylopentadecanolid, Ethylenbrassylat,
g) Ether wie Herbavert, Ambroxan,
sowie verschiedene weitere in der Parfümerie oft benutzte Komponenten wie Moschus- und Sandelholz-Riechstoffe, Indol, p-Menthan-8-thiol-3-on, Methyleugenol und Methylanthranilat.

Bemerkenswert ist ferner die Art und Weise, wie die Verbindungen der Struktur (I) die Geruchsnoten einer breiten Palette bekannter Kompositionen abrunden und harmonisieren, ohne jedoch in unangenehmer Weise zu dominieren.

Die einsetzbaren Anteile der erfindungsgemäßen Verbindungen (I) oder deren Gemische in Riechstoffkompositionen bewegen sich von etwa 1-70 Gew. %, bezogen auf die gesamte Mischung. Gemische der erfindungsgemäßen Verbindungen (I) sowie Kompositionen dieser Art können sowohl zur Parfümierung kosmetischer Präparate wie Lotionen, Cremes, Shampoos, Seifen, Salben, Pudern, Aerosolen, Zahnpasten, Mundwässern, Deodorantien als auch in der alkoholischen Parfümerie (z.B. Eau de Cologne, Eau de Toilette, Extraits) verwendet werden. Ebenso besteht eine Einsatzmöglichkeit zur Parfümierung technischer Produkte wie Wasch- und Reinigungsmittel, Weichspüler und Textilbehandlungsmittel. Zur Parfümierung dieser verschiedenen Produkte werden diesen die Kompositionen in einer olfaktorisch wirksamen Menge, insbesondere in einer Konzentration von 0,01-2 Gew. % - bezogen auf das gesamte Produkt - zugesetzt. Diese Werte sind jedoch keine beschränkenden Grenzwerte, da der erfahrenen Parfümeur auch mit noch geringeren Konzentrationen Effekte erzielen oder mit noch höheren Dosierungen neuartige Komplexe aufbauen kann.

### Beispiele

### Beispiel 1: Herstellung von 4,4-Diethoxy-2,6-dimethyl-heptan

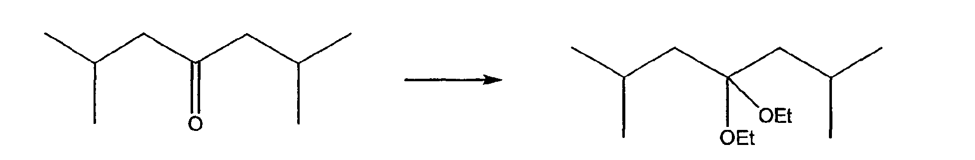

Ansatz:
1) 710,6g (4 mol) 2,6-Dimethyl-4-heptanon
2) 710,0 g (4,8 mol) Triethylorthoformiat
3)1,3 g (13,3 mmol) Schwefelsäure, konz.
4) 900 g Ethanol, MEK verg. 99%

Apparatur: 4L-Dreihalskolben, Rührer, Thermometer, Stickstoffatmosphäre.

Ausführung: Die Komponenten 1), 2), 4) und 3) wurden nacheinander in den Reaktionskolben gegeben und 7 Stunden bei Raumtemperatur unter Stickstoffatmosphäre gerührt. Der Reaktionsverlauf würde gaschromatographisch (GC) kontrolliert, nach 7 Stunden hatten sich 43% Produkt gebildet. Anschließend wurde durch Zugabe von 3,6 g Natriummethanolat-Lösung (30%ig in Methanol) neutralisiert. Am Rotationsverdampfer wurden im Wasserstrahlvakuum überschüssiges Ethanol und der gebildete Ameisensäureethylester abdestilliert. 730 g rohes Diethylketal des 2,6-Dimethyl-4-heptanons wurde an einer 20 cm Vigreuxkolonne destilliert. Die Hauptmenge von 227 g (Siedepunkt 48°C/0,09 mbar) mit einer GC-Reinheit von 98,4% wurde für die weitere Umsetzung verwendet.

### Beispiel 2: Herstellung von 4-(22-Diethoxy-ethyl)-2,6-dimethyl-4-ethoxy-heptan

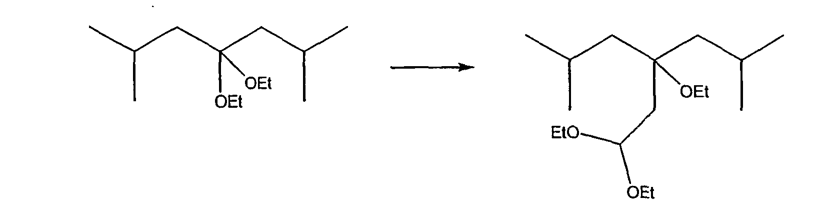

Ansatz:
1) 360,2 g (1,6 mol) 4,4-Diethoxy-2,6-dimethyl-heptan (hergestellt nach Beispiel 1)
2) 160 ml Zinkchloridlösung (10% in Essigester)
3) 138,2 g (1,92 mol) Ethylvinylether

Apparatur: 2L-Rührapparatur mit Thermometer, Rückflußkühler und Tropftrichter

Ausführung: Die Komponenten 1) und 2) wurden nacheinander in den Reaktionskolben eingewogen und unter Rühren auf 42°C erwärmt. Die Komponente 3 wurde innerhalb 1,5 Stunden unter Rühren kontinuierlich zudosiert. Es wurde noch 8 Stunden bei 40° C gerührt. Es konnte allerdings kein vollständiger Umsatz erreicht werden. 1,4% des Ausgangsmaterials hatten nicht reagiert. 37 % des Diethylketals hatten unter den Reaktionsbedingungen Ethanol eliminiert und den Enolether gebildet, der sich nicht mehr weiter umsetzte.

Aufarbeitung: Der Ansatz wurde in einen Scheidetrichter überführt und mit Wasser und Natriumhydrogencarbonatlösung neutral gewaschen. Die organische Phase wurde über Natriumsulfat getrocknet und am Rotationsverdampfer eingeengt.

Es wurden 376,3 g Rohprodukt mit einem Produkt-Gehalt von 35% (2 Isomere) erhalten. Diese wurden an einer Vigreuxkolonne destilliert. Im Hauptlauf wurden 112,3 g 4-(2,2-Diethoxy-ethyl)-2,6-dimethyl-4-ethoxy-heptan (Siedepunkt 73 - 84°C/0.08 mbar, GC-Reinheit 98,7%) (2 Isomere) erhalten.

Analytik: Das IR-Spektrum (Film zwischen NaCl) zeigte charakteristische Etherbanden bei 996, 1069, 1124 und 1157 cm⁻¹.

Das ¹H-NMR-Spektrum (in CDCl₃, 400 MHz) zeigte 4 Methylgruppen als Dubletts bei 1,2 ppm und 3 Methylgruppen als Tripletts bei 0,8 und 0,9 ppm. Die 2 CH₂-Gruppen gaben Signale zwischen 1,6 und 2,0 ppm als Multipletts von Dubletts. 3 CH₂-Gruppen aus Ethylresten gaben Quadrupletts bei 3,3; 3,5 und 3,6 ppm. Ein einzelnes Proton fand sich bei 4,7 ppm (Triplett, Acetal-Proton), begleitet von einem weniger intensiven Triplett für ein Isomeres. Die beiden verbleibenden Protonen an C-2 und C-6 vermuten wir bei 1,3 - 1,5 ppm, sie waren allerdings nicht als Nonetts erkennbar.

Geruchscharakteristik: Im Angeruch leicht fruchtig, beerig, Petroleum, grün, Schokolade, nach 24 Stunden am Riechstreifen war der Geruch schwach Cumin, fettig, holzig.

### Beispiel 3: Darstellung von 5-Methyl-3-isobutyl-2-hexenal im Gemisch mit 5-Methyl-3-isobutyl-3-hexenal

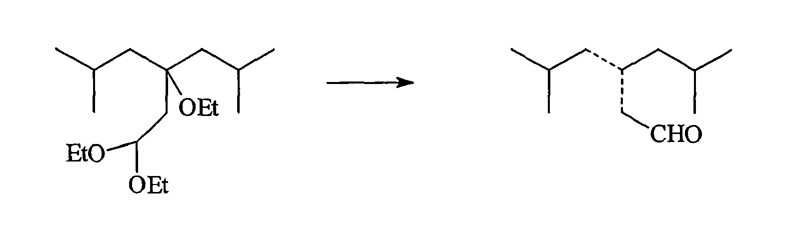

Ansatz:
1) 111,1 g (0,39 mol) 4-(2,2-Diethoxy-ethyl)-2,6-dimethyl-4-ethoxy-heptan
   (hergestellt nach Beispiel 2)
2) 67,3 g (1,46 mol) Ameisensäure
3) 17,9 g (0,26 mol) Natriumformiat
4) 28,6 g Wasser

Apparatur: 0,5 L Rührapparatur mit Thermometer, Rückflußkühler und Tropftrichter.

### Ausführung:

Die Komponenten 2), 3) und 4) wurden vorgelegt und unter Rühren auf Rückflußtemperatur (94°C) gebracht. Das nach Beispiel 2 hergestellte Acetal, die Komponente 1) wurde nun in 1 Stunde kontinuierlich zugetropft. Dabei fiel die Rückflußtemperatur auf 80°C ab. Es wurde noch 5 Stunden am Rückfluß 78 -62°C gekocht. Anschließend wurden noch einmal die gleiche Menge der Komponenten 2), 3) und 4) zugefügt und erneut 5 Stunden am Rückfluß gekocht.

Das Reaktionsgemisch wurde abgekühlt, in einen Scheidetrichter überführt und die wäßrige Phase abgetrennt. Die organische Phase wurde mit 500 ml Eiswasser gewaschen. Die Wasserphase wurde 3 mal mit Ether extrahiert. Die organischen Phasen wurden vereinigt und 1-mal mit Wasser, 2-mal mit Natriumhydrogencarbonatlösung, 2-mal mit Sodalösung und 1 mal mit Wasser gewaschen. Anschließend wurde über MgSO₄ getrocknet und am Rotationsverdampfer eingeengt. Es wurden 48,3 g Rohprodukt erhalten.

Die Destillation des Rohprodukts an einer 20 cm Vigreuxkolonne ergab 32 g Hauptlauf (Siedepunkt 54 - 55°C/0.06 mbar); die gaschromatographisch bestimmte Reinheit betrug 99%.

Das IR-Spektrum (Film zwischen NaCl) zeigte Carbonylschwingungsbanden bei 1676 und 1724 cm⁻¹ und 2 Banden bei 2765 cm⁻¹.

Das ¹H-NMR-Spektrum (400 MHz, in CDCl₃) war sehr komplex und wird wie folgt interpretiert: Die 12 Protonen der 4 Methylgruppen liegen alle zwischen 0,8 und 1,0 ppm. Zwischen 1,1 und 2,5 ppm liegen mehrere Signale und Gruppen, die teilweise zu starken Signalhaufen aufgespalten sind, so daß keine eindeutige Zuordnung getroffen werden kann. Als olefinisches Proton sind 3 Dubletts zwischen 5,8 und 6,0 ppm sichtbar in Kopplung mit ca. 4 Dubletts für das aldehydische Proton bei 9,9 und 10,0 ppm.

Geruchscharakteristik: Im Angeruch fettig, fruchtig, Citrus, Citral und im Nachgeruch nach 24 Stunden am Riechstreifen: ranzig, fettig, Farbe, Schweiß, Petroleum.

## Patentansprüche

1. Verwendung von Hexenal-Derivaten der allgemeinen Struktur (I) 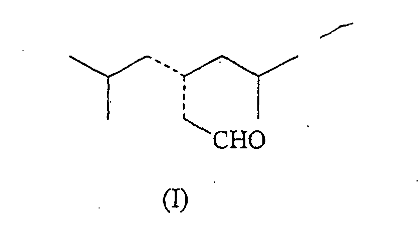 worin eine der strichlierten Bindungen eine C-C-Einfachbindung und die andere eine C=C-Doppelbindung bedeutet, mit der Maßgabe, daß die C=C-Doppelbindung Z- oder E-konfiguriert sein kann, sofern sie sich in Position C3/4 befindet, als Riechstoffe.

2. Verwendung gemäß Anspruch 1, wobei eine oder mehrere Verbindungen der allgemeinen Struktur (I) ein Riechstoff-Konzentrat bilden.

3. Riechstoff-Kompositionen mit einem Gehalt an einer oder mehreren Verbindungen der allgemeinen Struktur (I) 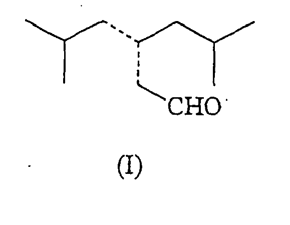 worin eine der strichlierten Bindungen eine C-C-Einfachbindung und die andere eine C=C-Doppelbindung bedeutet, mit der Maßgabe, daß die C=C-Doppelbindung Z- oder E-konfiguriert sein kann, sofern sie sich in Position C3/4 befindet, wobei die Verbindungen (I) in einer Menge von 1 bis 70 Gew.-% - bezogen auf die gesamte Komposition - enthalten sind.

## Claims

1. Use of hexenal derivatives of the general structure (I) 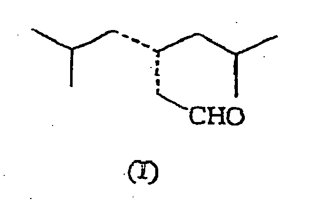 wherein one of the dashed-line bonds denotes a C-C single bond and the other denotes a C=C double bond, with the proviso that the C=C double bond may be Z-configured or E-configured, provided it is situated in position C3/4, as perfumes.

2. Use according to claim 1, wherein one or more compounds of the general structure (I) form a perfume concentrate.

3. Perfume compositions containing one or more compounds of the general structure (I) 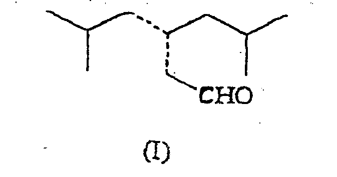 wherein one of the dashed-line bonds denotes a C-C single bond and the other denotes a C=C double bond, with the proviso that the C=C double bond may be Z-configured or E-configured, provided it is situated in position C3/4, wherein the compounds (I) are present in a quantity of 1 to 70 wt.% - relative to the total composition.

## Revendications

1. Utilisation de dérivés d'hexénal de structure générale (I) 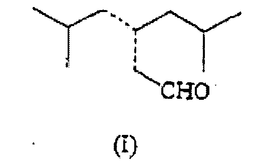 dans laquelle une des liaisons en pointillé signifie une liaison simple C-C et l'autre une double liaison C=C, à condition que la double liaison C=C puisse être configurée en Z ou en E dans la mesure où elle se trouve en position C3/4,
comme parfums.

2. Utilisation selon la revendication 1,
un ou plusieurs composés de la structure générale (I) formant un concentré de parfum.

3. Compositions de parfum avec une teneur en un ou plusieurs des composés de la structure générale (I) 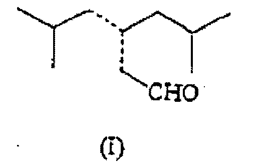 dans laquelle une des liaisons en pointillé signifie une liaison simple C-C et l'autre une double liaison C=C, à condition que la double liaison C=C puisse être configurée en Z ou en E dans la mesure où elle se trouve en position C3/4,
les composés (I) étant contenus en une quantité de 1 à 70 % en poids, par rapport à la composition totale.
